# EUROPEAN PATENT APPLICATION

(11) **EP 4 759 225 A1**
(43) Date of publication of application: **17.06.2026**
(21) Application number: 24870470.2
(22) Date of filing: 12.09.2024
(51) Int. Cl.: A61B 5/022

(54) **DETECTION MODULE AND ELECTRONIC DEVICE**

(30) Priority: 27.09.2023 CN 202311282947
(71) Applicant: HUAWEI TECHNOLOGIES CO., LTD., Shenzhen 518129 (CN)
(72) Inventor: DING, Li, Shenzhen, Guangdong 518129 (CN); CHEN, Qing, Shenzhen, Guangdong 518129 (CN); SONG, Nannan, Shenzhen, Guangdong 518129 (CN); LIU, Chuangchuang, Shenzhen, Guangdong 518129 (CN); CHEN, Baoyang, Shenzhen, Guangdong 518129 (CN); NI, Gang, Shenzhen, Guangdong 518129 (CN)
(74) Representative: Pfenning, Meinig & Partner mbB
(86) International application number: PCT/CN2024/118632
(87) International publication number: WO 2025/066926

(57) **Abstract**

A monitoring module and an electronic device are provided. The monitoring module includes a pressurization component (10) and a thin-film sensor (20). The pressurization component (10) is configured to apply pressure to a measured part. The thin-film sensor (20) is configured to measure a pulse wave signal at the measured part, and the thin-film sensor (20) is disposed between the pressurization component (10) and the measured part. The monitoring module and the electronic device can reduce difficulty in miniaturizing the electronic device.

## Description

This application claims priority to Chinese Patent Application No. 202311282947.1, filed with the China National Intellectual Property Administration on September 27, 2023 and entitled "MONITORING MODULE AND ELECTRONIC DEVICE", which is incorporated herein by reference in its entirety.

### TECHNICAL FIELD

Embodiments of this application relate to the field of terminal technologies, and in particular, to a monitoring module and an electronic device.

### BACKGROUND

Currently, an electronic device like a smart band or a smart watch is integrated with a blood pressure measurement function. The electronic device includes an air pump, an airbag, and a pressure sensor. The air pump is configured to inflate or deflate the airbag, the pressure sensor is configured to measure pressure in the airbag, and the airbag is configured to surround and compress arteries of a testee. However, it is difficult to miniaturize the electronic device. Therefore, how to miniaturize the electronic device becomes an urgent problem to be resolved.

### SUMMARY

Embodiments of this application provide a monitoring module and an electronic device, to reduce a difficulty of miniaturization.

A first aspect of this application provides a monitoring module, including a pressurization component and a thin-film sensor. The pressurization component is configured to apply pressure to a measured part. The thin-film sensor is configured to measure a pulse wave signal at the measured part, and the thin-film sensor is disposed between the pressurization component and the measured part.

When the monitoring module provided in this embodiment of this application monitors blood pressure, the pressurization component applies pressure to the measured part of a testee, so that blood vessels at the measured part are squeezed to different degrees, to generate pulse wave signals of different shapes and amplitudes, and the thin-film sensor can measure pulse wave signals in a region on the measured part, and can calculate the blood pressure and/or a heart rate of the testee based on the collected pulse wave signals. Because the pulse wave signal is collected via the thin-film sensor, signal collection can be decoupled from the pressurization component, so that a size constraint on the pressurization component can be reduced. This helps miniaturize the monitoring module. In addition, a change in the pulse wave signal is collected via the thin-film sensor, so that the monitoring module measures the pulse wave signal by using a mechanical method, and is not affected by factors such as ambient light, a skin color, and sweat, thereby improving monitoring accuracy. In addition, the thin-film sensor collects the pulse wave signals in the region on the measured part, to avoid measurement inaccuracy caused by a position deviation in single-point measurement, and further avoid a problem that a data scale is large and requirements on hardware complexity, hardware computing power, and an algorithm are high because a pressure sensor array is used to collect pulse wave signals in a region, and even consistency between a plurality of sensing units does not need to be considered, ensuring higher measurement efficiency and a more accurate result.

In a possible implementation, the monitoring module further includes a thin-film component. The thin-film component is disposed between the pressurization component and the thin-film sensor, or the thin-film sensor is disposed between the thin-film component and the pressurization component, or the thin-film component includes a first part and a second part, the first part is disposed between the pressurization component and the thin-film sensor, and the thin-film sensor is disposed between the first part and the second part.

According to the monitoring module provided in this embodiment of this application, the thin-film component is disposed, so that sensitivity of the thin-film sensor can be reduced, a signal output by the thin-film sensor due to a slight change in the measured part can be avoided, and a probability that the thin-film sensor operates unexpectedly can be reduced.

In a possible implementation, the monitoring module further includes a packaging component, and the thin-film sensor is disposed between the packaging component and the pressurization component and is shielded by the packaging component.

According to the monitoring module provided in this embodiment of this application, the thin-film sensor is disposed between the pressurization component and the packaging component and is shielded by the packaging component, so that the thin-film sensor can be protected from being worn, and erosion of oil stains, sweat stains, water vapor, and oxygen can be further isolated, to prolong a service life of the thin-film sensor.

In a possible implementation, the pressurization component includes an inflatable airbag, and the thin-film sensor is disposed between the measured part and the inflatable airbag.

According to the monitoring module provided in this embodiment of this application, the inflatable airbag is inflated or deflated, so that the inflatable airbag expands or contracts, to pressurize, depressurize or keep applying fixed pressure to the measured part, so as to ensure that the pulse wave signal at the measured part changes. In this way, the thin-film sensor can collect the pulse wave signal. In addition, because the pulse wave signal is collected via the thin-film sensor, a size constraint on the inflatable airbag is reduced, and the inflatable airbag can be designed to be narrower and shorter. This helps reduce a volume of the monitoring module.

In a possible implementation, the monitoring module further includes a binding component, the binding component is configured to limit a ring-shaped structure surrounding the measured part, and the pressurization component is disposed between the binding component and the thin-film sensor.

According to the monitoring module provided in this embodiment of this application, the pressurization component is disposed between the binding component and the thin-film sensor, so that the pressurization component can apply pressure to the measured part, to ensure that the pulse wave signal is generated due to squeezing of the blood vessels at the measured part.

In a possible implementation, the monitoring module further includes a barometric pressure sensor, and the barometric pressure sensor is configured to monitor pressure in the inflatable airbag.

According to the monitoring module provided in this embodiment of this application, the pressure in the inflatable airbag is monitored via the barometric pressure sensor, so that the blood pressure or the heart rate can be obtained based on a pressure change in the inflatable airbag, to correct the blood pressure or the heart rate obtained based on the pulse wave signal collected via the thin-film sensor, so as to further improve measurement accuracy. In addition, when the thin-film sensor cannot measure static force, it can be further ensured that the inflatable airbag can apply fixed pressure to the measured part, to implement a heart rate measurement function.

In a possible implementation, the pressurization component includes a watch buckle and a binding component, a first end of the binding component is fastened to the watch buckle, a second end of the binding component is in transmission connection to the watch buckle, the watch buckle and the binding component are configured to enclose a ring-shaped structure that surrounds the measured part and that has a variable inner diameter, and the thin-film sensor is disposed between the binding component and the measured part.

According to the monitoring module provided in this embodiment of this application, pressure is applied to the measured part via the pressurization component including the watch buckle and the binding component, so that a structural design of the monitoring module can be simplified. For example, parts such as an air pump and the barometric pressure sensor are not required. **In** addition, because the airbag is removed, a thickness of the pressurization component can be further reduced. **In** addition, the inner diameter of the ring-shaped structure limited by the pressurization component is variable, so that the monitoring module has an automatic tightening function, and measurement effect is not affected by an initial wearing status of a user, thereby avoiding a measurement error caused by excessively loose wearing or excessively tight wearing.

In a possible implementation, the thin-film sensor is of a strip-shaped structure, and a length direction of the thin-film sensor is parallel to a length direction of the pressurization component.

According to the monitoring module provided in this embodiment of this application, the length direction of the thin-film sensor is parallel to the length direction of the pressurization component, so that it can be ensured that the thin-film sensor is always opposite to arterial vessels at the measured part, to collect the pulse wave signal. In addition, measured parts of different testees can be further adapted to, to improve universality of the monitoring module.

In a possible implementation, the thin-film sensor is one of the following: a piezoelectric thin-film sensor, a piezoresistive thin-film sensor, a piezocapacitive thin-film sensor, an ionic-electronic thin-film sensor, or a triboelectric thin-film sensor. The pulse wave signal can be collected by using these types of thin-film sensors.

In a possible implementation, the thin-film sensor includes a first electrode layer, a sensing layer, and a second electrode layer that are stacked, one of the first electrode layer and the second electrode layer is disposed between the pressurization component and the sensing layer, and the other is disposed between the sensing layer and the measured part.

One of the first electrode layer and the second electrode layer of the thin-film sensor in the monitoring module provided in this embodiment of this application is disposed between the pressurization component and the sensing layer, and the other is disposed between the sensing layer and the measured part, so that it can be ensured that the pulse wave signal is collected when the blood vessel at the measured part generates the pulse wave signal.

A second aspect of this application provides an electronic device, including the monitoring module according to any implementation of the first aspect.

According to the electronic device provided in this embodiment of this application, the monitoring module in the first aspect is used, so that difficulty in miniaturizing the electronic device is reduced, and the electronic device can be miniaturized. In addition, because the monitoring module collects a pulse wave signal via a thin-film sensor, accuracy of monitoring a heart rate by the electronic device can be improved.

### BRIEF DESCRIPTION OF DRAWINGS

FIG. 1 is a diagram of a structure of a first electronic device according to an embodiment of this application;
FIG. 2 is a block diagram of a structure of the electronic device shown in FIG. 1;
FIG. 3 is a cross-sectional view of fitting between a thin-film sensor and a pressurization component in FIG. 1;
FIG. 4 is a diagram of a structure of a second electronic device according to an embodiment of this application;
FIG. 5 is a cross-sectional view of the electronic device in FIG. 4;
FIG. 6 is a partial cross-sectional view of a third electronic device according to an embodiment of this application;
FIG. 7 is a partial cross-sectional view of a fourth electronic device according to an embodiment of this application;
FIG. 8 is a partial cross-sectional view of a fifth electronic device according to an embodiment of this application; and
FIG. 9 is a diagram of a structure of a sixth electronic device according to an embodiment of this application.

### Descriptions of reference numerals:

10: pressurization component;
20: thin-film sensor; 21: first electrode layer; 22: sensing layer; 23: second electrode layer;
30: binding component; 31: watch body; 32: first watchband; 33: second watchband;
40: connection line;
50: barometric pressure sensor;
60: watch buckle;
70: thin-film component;
80: packaging component;
81: controller; 82: air pump; and 83: exhaust valve.

### DESCRIPTION OF EMBODIMENTS

Currently, an electronic device like a smart band, a smart watch, or a watch-type sphygmomanometer is integrated with a blood pressure measurement function, a heart rate measurement function, and the like. The electronic device includes an air pump, an airbag, and a pressure sensor. The air pump is configured to inflate or deflate the airbag, and the pressure sensor is configured to measure pressure in the airbag. Arterial vessels in a wrist are pressed through expansion of the airbag, a pulsation amplitude of the vessel also changes in a change process of external pressure, and the change affects air pressure inside the airbag. In an entire airbag pressurization process, the pressure sensor detects a pulse wave oscillating envelope signal that first increases and then decreases, and determines blood pressure based on the envelope signal. In addition, the electronic device measures a heart rate through photoplethysmography (PPG). The PPG is a method for illuminating light into skin and measuring light scattering generated due to blood flow. The method is based on the following operating principle: When a hemodynamic parameter, for example, a blood pulse rate (heart rate) or a blood volume (cardiac output), changes, light entering a human body undergoes foreseeable scattering. The scattered light is received by a photosensor and converted into electrical signals, and heart rate data of a testee can be obtained by analyzing periodicities of the obtained electrical signals.

However, to obtain a complete pulse wave oscillating envelope signal, a sufficient and effective press-fitting area needs to be provided when the airbag expands. Consequently, a width of the airbag is large, a volume of the electronic device is large, and miniaturization is difficult. In addition, the airbag needs to press a radial arterial vessel and an ulnar arterial vessel in the wrist, and a pressure upper limit of the airbag is large. Moreover, it is very difficult for an airbag of a standard size to cover people of all wrist circumferences. Therefore, a plurality of airbags need to be provided, and costs are increased.

In addition, because PPG measurement is based on an optical principle, all factors that affect intensity of the scattered light may cause a measurement error. For example, a dark skin color has a higher light absorption rate, which causes scattered light received by the photosensor to be reduced and a large error. For another example, when the wrist is worn loosely, a gap between the wrist and a PPG module is large, and a large amount of ambient light is introduced to superimpose with a scattered optical signal and cause impact. In addition, displacement of the sensor on the wrist caused by motion also leads to a deviation in the scattered optical signal, and results in the measurement error.

In view of this, embodiments of this application provide a monitoring module and an electronic device, to reduce difficulty in miniaturizing the electronic device. In addition, accuracy of measuring a heart rate and/or blood pressure can be further improved. In addition, a requirement of the electronic device on computing power can be further reduced, to improve feasibility of the electronic device.

The following describes implementations of the monitoring module and the electronic device provided in embodiments of this application.

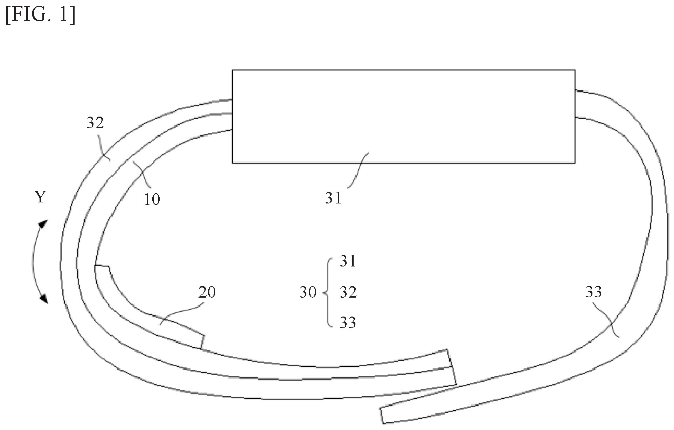

FIG. 1 is a diagram of a structure of a first electronic device according to an embodiment of this application. Refer to FIG. 1. The electronic device in this embodiment of this application includes a monitoring module. The monitoring module is configured to monitor blood pressure and/or a heart rate of a testee.

The electronic device provided in this embodiment of this application may include but is not limited to a smart band, a smart watch, a watch, a watch-type sphygmomanometer, a sphygmomanometer, or the like. In this embodiment of this application, an example in which the smart watch is the electronic device is used for description.

Still refer to FIG. 1. The monitoring module includes a pressurization component 10 and a thin-film sensor 20. The pressurization component 10 is configured to apply pressure to a measured part (not shown in the figure) of the testee. The thin-film sensor 20 is configured to measure a pulse wave signal at the measured part, and the thin-film sensor 20 is disposed between the pressurization component 10 and the measured part.

The thin-film sensor 20 is a flexible sheet-like sensor whose thickness is less than a preset thickness. For example, in this embodiment of this application, the thin-film sensor 20 is a flexible sheet-like sensor whose thickness is less than 0.5 mm.

With reference to FIG. 1, in a process in which the electronic device measures the measured part of the testee, the pressurization component 10 applies pressure to the measured part of the testee, so that blood vessels at the measured part are squeezed to different degrees, to generate pulse wave signals of different shapes and amplitudes, and the thin-film sensor 20 can measure pulse wave signals in a region on the measured part, and can calculate the blood pressure and/or the heart rate of the testee based on the collected pulse wave signals. The pressurization component 10 applies a plurality of different pressure values to the measured part, so that an envelope of the pulse wave signal changes, and the blood pressure value can be calculated based on the envelope change. The pressurization component 10 applies fixed pressure to the measured part, to collect statistics about frequencies of the pulse wave signal, and the heart rate value can be calculated based on the frequencies of the pulse wave signal.

In this embodiment of this application, the thin-film sensor 20 collects the pulse wave signal at the measured part, and the pressurization component 10 provides the pressure applied to the measured part, so that the pressurization component 10 can be decoupled from pulse wave signal collection. Therefore, a size constraint on the pressurization component 10 is reduced, and the pressurization component 10 can be designed to be narrower and shorter, so that a volume of the pressurization component 10 can be reduced, thereby helping miniaturize the monitoring module.

In this embodiment of this application, the pressurization component 10 applies the fixed pressure to the measured part, and the thin-film sensor 20 collects the frequency of the pulse wave signal, so that the monitoring module measures the pulse wave signal by using a mechanical method. Compared with a related technology in which a heart rate is measured by using a photoplethysmography method, the monitoring module provided in this embodiment of this application can measure the heart rate without being affected by factors such as ambient light, a skin color, and sweat, thereby improving monitoring accuracy.

In this embodiment of this application, the thin-film sensor 20 collects the pulse wave signals in the region on the measured part, to avoid measurement inaccuracy caused by a position deviation in single-point measurement. In addition, a problem that a data scale is large and requirements on hardware complexity, hardware computing power, and an algorithm are high because a pressure sensor array is used to collect pulse wave signals in a region can be further avoided, and even consistency maintenance between a plurality of sensing units does not need to be considered, ensuring higher measurement efficiency and a more accurate result.

Still refer to FIG. 1. In a length direction (for example, a Y direction in FIG. 1) of the pressurization component 10, a length of the thin-film sensor 20 is less than a length of the pressurization component 10. Certainly, the length of the thin-film sensor 20 may alternatively be equal to the length of the pressurization component 10.

It should be noted that, in a measurement process, the pressurization component 10 covers two arteries at the measured part, and the pressurization component 10 drives the two arteries to vibrate, so that skin at the measured part vibrates, and a plurality of signal sources are generated, where at least one of the plurality of signal sources may be a clutter signal that interferes with measurement of the thin-film sensor 20. Therefore, if the length of the thin-film sensor 20 is excessively long, the thin-film sensor 20 may be subject to interference of the clutter signal, and measurement effect of the thin-film sensor 20 is reduced. Certainly, if the length of the thin-film sensor 20 is excessively short, the thin-film sensor 20 may not cover the artery, and cannot measure the pulse wave signal.

In some embodiments, still as shown in FIG. 1, the thin-film sensor 20 may be connected to an outer surface of the pressurization component 10, so that the thin-film sensor 20 can be fastened to the pressurization component 10, and the thin-film sensor 20 can be disposed between the pressurization component 10 and the measured part. The thin-film sensor 20 may be attached to the surface of the pressurization component 10 through bonding, hot pressing, or the like.

In this embodiment of this application, a type of the thin-film sensor 20 may include but is not limited to a piezoelectric thin-film sensor, a piezoresistive thin-film sensor, a piezocapacitive thin-film sensor, an ionic-electronic thin-film sensor, or a triboelectric thin-film sensor. A sensing material of the piezoelectric thin-film sensor may include an inorganic piezoelectric material like aluminum nitride, zinc oxide, or lead zirconate titanate, and an organic piezoelectric material like polyvinylidene fluoride. A sensing material of the piezoresistive thin-film sensor may include carbon black, a carbon nanotube, or the like. A sensing material of the piezocapacitive thin-film sensor may include an elastic material like polydimethylsiloxane, Ecoflex, or a hydrogenated styrene-butadiene block copolymer. A sensing material of the ionic-electronic thin-film sensor may include ionic liquid, ionic gel, or the like. A sensing material of the triboelectric thin-film sensor may include polyethylene terephthalate, polytetrafluoroethylene, or the like.

In some possible implementations, with reference to FIG. 1, the thin-film sensor 20 may be of a strip-shaped structure, and a length direction of the thin-film sensor 20 is parallel to the length direction of the pressurization component 10. In this way, it can be ensured that the thin-film sensor 20 is always opposite to the arterial vessels at the measured part. This not only can ensure that the thin-film sensor 20 collects the pulse wave signal, but also can reduce the length of the thin-film sensor 20, thereby reducing costs of the thin-film sensor 20. In addition, measured parts of different testees can be further adapted to, to improve universality of the monitoring module.

A specific shape of the thin-film sensor 20 is not limited herein. For example, the thin-film sensor 20 may be in a rectangular shape.

It should be noted that, in addition to the strip-shaped structure, the thin-film sensor 20 may alternatively be of a circular structure, a square structure, or the like.

In some possible implementations, with reference to FIG. 1, the pressurization component 10 includes an inflatable airbag. The thin-film sensor 20 is disposed between the measured part and the inflatable airbag, and the inflatable airbag is of a strip-shaped structure. In the measurement process, the inflatable airbag covers the artery at the measured part, and the inflatable airbag is inflated or deflated, so that the inflatable airbag expands or contracts, to pressurize, depressurize or keep applying fixed pressure to the measured part, so as to ensure that the pulse wave signal at the measured part changes. In addition, the thin-film sensor 20 can collect the pulse wave signal.

Correspondingly, because the pulse wave signal is collected via the thin thin-film sensor 20 and the inflatable airbag is configured to apply pressure to the measured part, the inflatable airbag is decoupled from pulse wave signal collection. Therefore, a size constraint on the inflatable airbag is reduced, and the inflatable airbag can be designed to be narrower and shorter. This helps reduce the volume of the monitoring module.

A specific material of the inflatable airbag is not limited herein. The material of the inflatable airbag may include a polymer material like a thermoplastic polyurethane elastomer, a thermoplastic elastomer, polyurethane, or nitrile rubber.

The inflatable airbag may be of a single-layer structure, or may be of a multi-layer structure. This is not limited herein.

The inside of the inflatable airbag is a closed structure, and the inflatable airbag may be connected to the outside via one or more air nozzles (not shown in the figure), so that external gas enters and exits the inside of the inflatable airbag, to implement expansion and contraction of the inflatable airbag. How to enable the gas to enter and exit the inflatable airbag is not limited herein.

In some possible implementations, with reference to FIG. 1, the monitoring module may further include a binding component 30. The binding component 30 is configured to limit a ring-shaped structure surrounding the measured part, and the pressurization component 10 is disposed between the binding component 30 and the thin-film sensor 20. In this way, the pressurization component 10 can apply pressure to the measured part, to ensure that the pulse wave signal is generated due to squeezing of the blood vessels at the measured part.

It may be understood that, with reference to FIG. 1, it can be learned that the pressurization component 10 is of a strip-shaped structure, the pressurization component 10 is the inflatable airbag, and the inflatable airbag cannot surround the measured part. In this case, the inflatable airbag cannot apply pressure to the measured part. Therefore, in this embodiment of this application, the pressurization component 10 is disposed between the thin-film sensor 20 and the binding component 30, so that the pressurization component 10 can apply pressure to the measured part in the measurement process, thereby ensuring that the thin-film sensor 20 can measure the pulse wave signal.

A specific structure of the binding component 30 is not limited herein, provided that a requirement that the pressurization component 10 can apply pressure to the measured part is met.

For example, still refer to FIG. 1. The binding component 30 may include a watch body 31, a first watchband 32, and a second watchband 33. A first end of the first watchband 32 and a first end of the second watchband 33 are respectively connected to two opposite ends of the watch body 31, and a second end of the first watchband 32 is detachably connected to a second end of the second watchband 33. The pressurization component 10 is disposed on an inner side of one of the first watchband 32 and the second watchband 33. For example, as shown in FIG. 1, the pressurization component 10 is disposed on the inner side of the first watchband 32, so that the pressurization component 10 is disposed between the first watchband 32 and the measured part. In the measurement process, the second end of the first watchband 32 is connected to the second end of the second watchband 33, so that the binding component 30 forms the ring-shaped structure surrounding the measured part, the pressurization component 10 is disposed between the binding component 30 and the measured part, and further the pressurization component 10 can apply pressure to the measured part.

Still refer to FIG. 1. The length of the pressurization component 10 is equal to a length of the first watchband 32. However, the length of the pressurization component 10 may alternatively be less than the length of the first watchband 32.

In some embodiments, a width of the pressurization component 10 is equal to a width of the first watchband 32. Certainly, the width of the pressurization component 10 may alternatively be less than the width of the first watchband 32.

In this embodiment of this application, a specific structure of the watch body 31 is not limited herein. For example, the watch body 21 may include a housing and a display panel. The first end of the first watchband 32 and the first end of the second watchband 33 are respectively connected to two opposite ends of the housing, and the display panel is configured to display to-be-displayed information, for example, text or an image.

It should be noted that the binding component 30 may be of another structure in addition to including the watch body 31, the first watchband 32, and the second watchband 33. For example, the binding component 30 is of a strip-shaped strip component (not shown in the figure), the pressurization component 10 is disposed on an inner side of the strip component, and the strip component is configured to enclose the ring-shaped structure surrounding the measured part. Alternatively, in some embodiments, the binding component 30 is of a ring-shaped component (not shown in the figure), and the pressurization component 10 is disposed on an inner side of the ring-shaped component and is disposed between the ring-shaped component and the measured part.

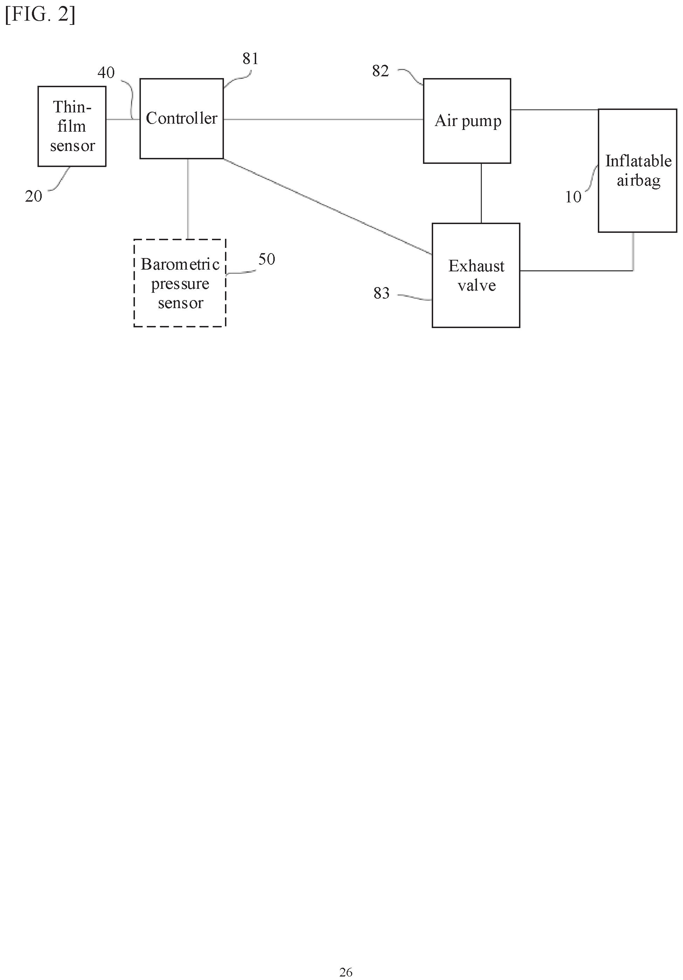

FIG. 2 is a block diagram of a structure of the electronic device shown in FIG. 1. In some possible implementations, as shown in FIG. 2, the electronic device may further include an air pump 82 and an exhaust valve 83. The inflatable airbag may be separately connected to the air pump 82 and the exhaust valve 83 via the two air nozzles, and the exhaust valve 83 is connected to the air pump. The air pump 82 fits the exhaust valve 83 to inflate or deflate the inflatable airbag, so that the inflatable airbag expands and contracts, to meet a requirement of applying pressure to or releasing pressure from the measured part.

In some embodiments, the air pump 82 and the exhaust valve 83 may be disposed inside the watch body 31 (not shown in the figure), to improve integration of the electronic device.

In some possible implementations, still as shown in FIG. 2, the electronic device may further include a controller 81. The controller 81 is electrically connected to the thin-film sensor 20, the air pump 82, and the exhaust valve 83 separately. The controller 81 may be configured to control the air pump 82 and the exhaust valve 83, to inflate or deflate the inflatable airbag. In addition, the controller 81 may further calculate the blood pressure and/or the heart rate based on the pulse wave signal collected by the thin-film sensor 20.

In some embodiments, the controller 81 may be disposed inside the watch body 31 (not shown in the figure), to improve the integration of the electronic device.

In some possible implementations, still as shown in FIG. 2, the monitoring module may further include a barometric pressure sensor 50, and the barometric pressure sensor 50 is configured to monitor pressure in the inflatable airbag. Correspondingly, the pressure in the inflatable airbag is monitored via the barometric pressure sensor 50, so that the blood pressure or the heart rate can be obtained based on a pressure change in the inflatable airbag, to correct the blood pressure or the heart rate obtained based on the pulse wave signal collected via the thin-film sensor 20, so as to further improve measurement accuracy. In addition, when the thin-film sensor 20 cannot measure static force, it can be further ensured that the inflatable airbag can apply fixed pressure to the measured part, to implement a heart rate measurement function.

It may be understood that, when the thin-film sensor 20 can monitor only dynamic force, if there is no barometric pressure sensor 50, when the inflatable airbag applies the fixed pressure to the measured part, the pressure collected by the thin-film sensor 20 is zero. In this case, the frequency of the pulse wave signal under the fixed pressure cannot be measured, and consequently, the heart rate cannot be measured. Therefore, when the thin-film sensor 20 cannot measure the static force, the pressure in the inflatable airbag is monitored via the barometric pressure sensor 50, so that it can be ensured that the inflatable airbag can apply the fixed pressure to the measured part, to implement heart rate monitoring.

A specific mounting position of the barometric pressure sensor 50 is not limited herein. In an embodiment, the barometric pressure sensor 50 may be disposed inside the inflatable airbag (not shown in the figure). Alternatively, in an embodiment, the barometric pressure sensor 50 may be disposed inside the watch body 31 (not shown in the figure).

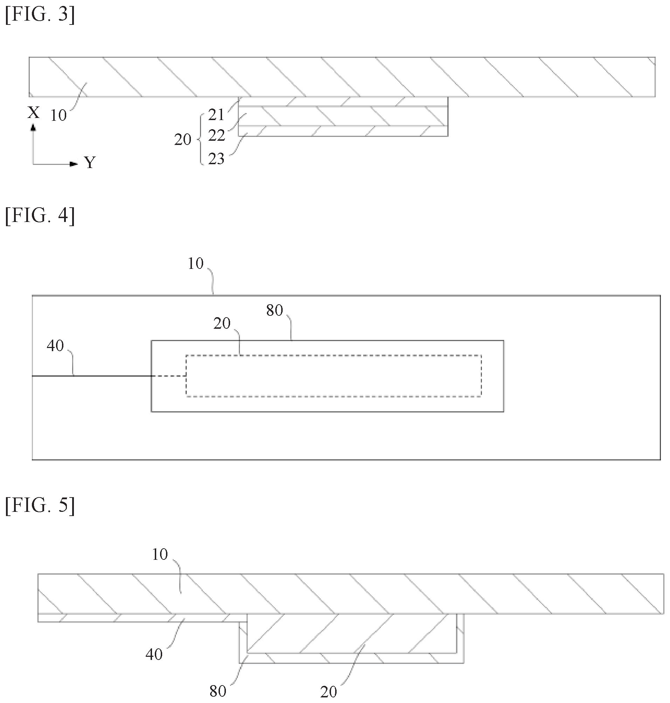

FIG. 3 is a cross-sectional view of fitting between the thin-film sensor and the pressurization component in FIG. 1. In some possible implementations, the thin-film sensor 20 may include a first electrode layer 21, a sensing layer 22, and a second electrode layer 23 that are stacked. One of the first electrode layer 21 and the second electrode layer 23 is disposed between the pressurization component 10 and the sensing layer 22, and the other is disposed between the sensing layer 22 and the measured part. For example, as shown in FIG. 3, the first electrode layer 21 is disposed between the pressurization component 10 and the sensing layer 22, and the second electrode layer 23 is disposed between the sensing layer 22 and the measured part.

In the process in which the pressurization component 10 applies pressure to the measured part, the second electrode layer 23 is configured to be close to the skin at the measured part. When the sensing layer 22 is subject to the external pressure, distribution of charged particles, for example, electrons and ions, in a material changes, the change is transmitted from the electrode layer to generate a change of an electrical signal, and measurement of a pressure value by the thin-film sensor 20 is completed through one-to-one mapping between the electrical signal and the external pressure value. In this embodiment of this application, the external pressure value may be understood as pressure applied by the blood vessel to the thin-film sensor 20 under pressure applied by the pressurization component 10.

In some embodiments, still as shown in FIG. 3, in a thickness direction (for example, an X direction in FIG. 3) of the thin-film sensor 20, a thickness of the pressurization component 10 is equal to a thickness of the thin-film sensor 20. Certainly, the thickness of the pressurization component 10 may alternatively be greater than or less than that of the thin-film sensor 20 (not shown in the figure).

In some embodiments, still as shown in FIG. 3, in the thickness direction of the thin-film sensor 20, thicknesses of the first electrode layer 21 and the second electrode layer 23 are the same, and a thickness of either the first electrode layer 21 or the second electrode layer 23 is equal to half a thickness of the sensing layer 22. In this way, the thickness of the thin-film sensor 20 can be reduced. This facilitates miniaturization of the monitoring module.

The monitoring module may further include a connection line 40. With reference to FIG. 2, and still as shown in FIG. 4 and FIG. 5, one end of the connection line 40 is electrically connected to the thin-film sensor 20, the other end of the connection line 40 is configured to be electrically connected to the controller 81 of the electronic device, and the connection line 40 may transmit, to the controller 81, the pulse wave signal collected by the thin-film sensor 20.

The connection line 40 may be electrically connected to at least one of the thin-film sensor 20 and the controller 81 through insertion, welding, conductive adhesive bonding, or the like.

A specific type of the connection line 40 is not limited herein. The type of the connection line 40 may include but is not limited to an enameled wire, a rubber conducting wire, and a flexible printed circuit (flexible printed circuit, FPC for short) flat cable. In addition, a conductor material of the connection line 40 may include but is not limited to gold, silver, copper, aluminum, graphite, a silver nanowire, or the like.

Still refer to FIG. 4 and FIG. 5. The connection line 40 is disposed outside the pressurization component 10. Certainly, the connection line 40 may alternatively be disposed inside the pressurization component 10 (not shown in the figure).

Still refer to FIG. 4 and FIG. 5. The connection line 40 is connected to the pressurization component 10. Certainly, the connection line 40 may alternatively be arranged on the outer side of the pressurization component 10 in a form of a jump wire. In other words, the connection line 40 may be connected to or not connected to the pressurization component 10.

How the connection line 40 is connected to the pressurization component 10 is not limited herein. For example, the connection line 40 may be connected to the pressurization component 10 through surface attachment, deposition, or the like.

In some possible implementations, with reference to FIG. 4 and FIG. 5, the monitoring module may further include a packaging component 80, and the thin-film sensor 20 is disposed between the packaging component 80 and the pressurization component 10 and is shielded by the packaging component 80. In this way, the thin-film sensor 20 can be protected from being worn, and erosion of oil stains, sweat stains, water vapor, and oxygen can be further isolated, to prolong a service life of the thin-film sensor 20.

The packaging component 80 may be attached to the surface of the pressurization component 10 through bonding, hot pressing, or the like, to cover the thin-film sensor 20.

A material of the packaging component 80 may include but is not limited to a dense insulation material like a thermoplastic polyurethane elastomer, a thermoplastic elastomer, polyurethane (PU), nitrile rubber, or polytetrafluoroethylene.

Still refer to FIG. 4 and FIG. 5. Because the connection line 40 is disposed on the outer side of the pressurization component 10, the packaging component 80 also covers a part of the connection line 40. Certainly, when the connection line 40 is disposed inside the pressurization component 10, the packaging component 80 does not cover the connection line 40 (not shown in the figure).

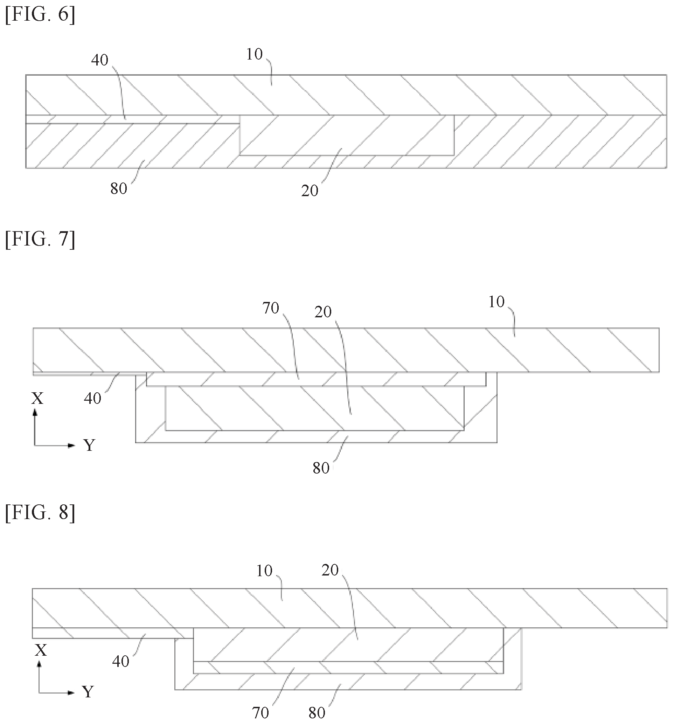

Still refer to FIG. 4 and FIG. 5. An area of the packaging component 80 is greater than an area of the thin-film sensor 20, and is less than an area of the pressurization component 10. In other words, the packaging component 80 not only covers the entire thin-film sensor 20, but also covers a part of the pressurization component 10. Certainly, in some embodiments, the packaging component 80 may alternatively cover an entire surface of the pressurization component 10. For example, FIG. 6 is a partial cross-sectional view of a third electronic device according to an embodiment of this application. Alternatively, in some embodiments, the packaging component 80 may cover only a surface of the thin-film sensor 20 (not shown in the figure).

FIG. 7 is a partial cross-sectional view of a fourth electronic device according to an embodiment of this application. A difference between FIG. 7 and FIG. 5 lies in that the monitoring module may further include a thin-film component 70, and the thin-film component 70 is disposed between the pressurization component 10 and the thin-film sensor 20. In this way, sensitivity of the thin-film sensor 20 can be reduced, a signal output by the thin-film sensor 20 due to a slight change in the measured part can be avoided, and a probability that the thin-film sensor 20 operates unexpectedly can be reduced.

There is natural movement at the measured part, for example, unconscious skin vibration of the wrist, the vibration also causes the thin-film sensor 20 to output a signal, but the signal is meaningless. Therefore, the sensitivity of the thin-film sensor 20 needs to be controlled within a specific range. If the sensitivity of the thin-film sensor 20 is excessively high, the thin-film sensor 20 outputs the signal due to the natural movement at the measured part. If the sensitivity of the thin-film sensor 20 is excessively low, the measurement accuracy is affected. Therefore, the sensitivity of the thin-film sensor 20 is reduced via the thin-film component 70, so that the sensitivity of the thin-film sensor 20 can be controlled within the specific range.

In this embodiment of this application, a material of the thin-film component 70 may include but is not limited to polyethylene naphthalate, polyethylene terephthalate, or the like.

In this embodiment of this application, a specific thickness of the thin-film component 70 is not limited herein. For example, the thickness of the thin-film component 70 may be 100 µm. A larger thickness of the thin-film component 70 indicates a larger reduction amplitude of the sensitivity of the thin-film sensor 20.

Still refer to FIG. 7. An area of the thin-film component 70 is greater than the area of the thin-film sensor 20. In other words, in the thickness direction (for example, an X direction in FIG. 7) of the thin-film sensor 20, projection of the thin-film component 70 covers projection of the thin-film sensor 20. Certainly, the area of the thin-film component 70 may alternatively be equal to the area of the thin-film sensor 20 (not shown in the figure). Therefore, the area of the thin-film component 70 is greater than or equal to the area of the thin-film sensor 20, to avoid affecting the measurement effect of the thin-film sensor 20 due to bending of the thin-film component 70, and reduce the sensitivity of the thin-film sensor 20.

Still refer to FIG. 7. The packaging component 80 covers both the thin-film sensor 20 and the thin-film component 70. In other words, the packaging component 80 simultaneously shields the thin-film component 70 and the thin-film sensor 20. Certainly, the packaging component 80 may alternatively shield a part of the thin-film component 70 (not shown in the figure) while covering the thin-film sensor 20.

It should be noted that, because the thin-film sensor 20 is the flexible sheet-like sensor whose thickness is less than 0.5 mm, the thin-film sensor 20 preferably fits a rigid object, that is, the thin-film sensor 20 fits an object whose rigidness is greater than rigidness of the thin-film sensor 20. With reference to FIG. 1, it can be learned that two sides of the thin-film sensor 20 are respectively the inflatable airbag and the skin at the measured part, and because rigidness of both the inflatable airbag and the skin at the measured part is less than the rigidness of the thin-film sensor 20, a clutter signal that interferes with the thin-film sensor 20 is generated and/or a signal output by the thin-film sensor 20 is unstable. Therefore, if the thin-film sensor 20 fits a soft object, the measurement effect is reduced. It can be learned that the sensitivity of the thin-film sensor 20 can be reduced, and the measurement effect can be improved by disposing any object whose rigidness is greater than that of the thin-film sensor 20 on at least one side of the thin-film sensor 20.

In conclusion, the thin-film component 70 is disposed between the thin-film sensor 20 and the pressurization component 10, to control the sensitivity of the thin-film sensor 20 within the specific range. FIG. 8 is a partial cross-sectional view of a fifth electronic device according to an embodiment of this application. In some embodiments, as shown in FIG. 8, the thin-film sensor 20 may alternatively be disposed between the pressurization component 10 and the thin-film component 70, so that the sensitivity of the thin-film sensor 20 can also be controlled within the specific range.

Alternatively, in some embodiments, the thin-film component 70 may alternatively include a first part and a second part (not shown in the figure), the first part is disposed between the pressurization component 10 and the thin-film sensor 20, and the thin-film sensor 20 is disposed between the first part and the second part, so that the sensitivity of the thin-film sensor 20 can also be controlled within the specific range. Thicknesses of the first part and the second part may be equal, or the thickness of the first part may be greater than the thickness of the second part, or the thickness of the first part may be less than the thickness of the second part.

Certainly, in addition to implement sensitivity reduction of the thin-film sensor 20 via the thin-film component 70, if an object whose rigidness is greater than the rigidness of the thin-film sensor 20 is disposed on one side of the thin-film sensor 20, the thin-film component 70 may alternatively be removed. For example, rigidness of the inflatable airbag is greater than the rigidness of the thin-film sensor 20, and the thin-film component 70 may alternatively be removed while the sensitivity of the thin-film sensor 20 is reduced via the inflatable airbag. Alternatively, in some embodiments, rigidness of the packaging component 80 is greater than the rigidness of the thin-film sensor 20, and the thin-film component 70 may alternatively be removed while the rigidness of the thin-film sensor 20 is reduced via the packaging component 80.

In the foregoing content, the inflatable airbag fits the binding component 30, to apply pressure to the measured part. Certainly, the binding component may alternatively be removed, and the pressurization component 10 can apply pressure to the measured part. In some possible implementations, the pressurization component 10 may alternatively include an inflatable airbag and a variable-diameter structure (not shown in the figure). The inflatable airbag is of a ring-shaped structure, and the variable-diameter structure is configured to change an outer diameter of the inflatable airbag, so that the inflatable airbag fits different testees.

Alternatively, in some possible implementations, the pressurization component 10 may include a first structure (not shown in the figure), a second structure (not shown in the figure), and an inflatable airbag of a strip-shaped structure (not shown in the figure), the first structure and the second structure are respectively disposed at two opposite ends of the inflatable airbag, and the first structure and the second structure are detachably connected. In the measurement process, the first structure is connected to the second structure, so that the inflatable airbag forms a ring-shaped structure surrounding the measured part. Therefore, the thin-film sensor 20 is disposed between the inflatable airbag and the measured part, and the thin-film sensor 20 can collect the pulse wave signal.

The first structure may be detachably connected to the second structure through bonding or clamping.

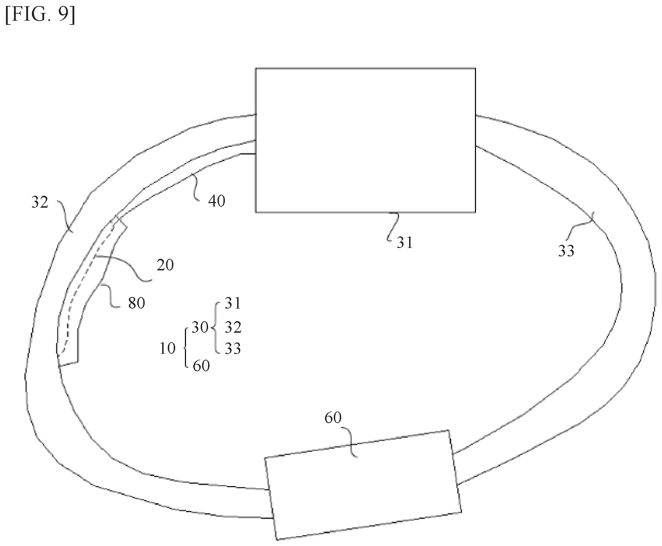

FIG. 9 is a diagram of a structure of a sixth electronic device according to an embodiment of this application. A difference between FIG. 9 and FIG. 1 lies in that the pressurization component 10 includes a watch buckle 60 and a binding component 30, a first end of the binding component 30 is fastened to the watch buckle 60, a second end of the binding component 30 is in transmission connection to the watch buckle 60, the watch buckle 60 and the binding component 30 are configured to enclose a ring-shaped structure that surrounds the measured part and that has a variable inner diameter, and the thin-film sensor 20 is disposed between the binding component 30 and the measured part.

Correspondingly, pressure is applied to the measured part via the pressurization component 10 including the watch buckle 60 and the binding component 30, so that a structural design of the monitoring module can be simplified. For example, parts such as the air pump 82 and the barometric pressure sensor 50 are not required. In addition, because the airbag is removed, the thickness of the pressurization component 10 can be further reduced. In addition, the inner diameter of the ring-shaped structure limited by the pressurization component 10 is variable, so that the monitoring module has an automatic tightening function, and the measurement effect is not affected by an initial wearing status of a user, thereby avoiding a measurement error caused by excessively loose wearing or excessively tight wearing.

Still refer to FIG. 9. The binding component 30 includes a watch body 31, a first watchband 32, and a second watchband 33. A first end of the first watchband 32 and a first end of the second watchband 33 are respectively connected to two opposite ends of the watch body 31, and a second end of the first watchband 32 and a second end of the second watchband 33 are separately connected to the watch buckle 60. The thin-film sensor 20 is disposed on an inner side of one of the first watchband 32 and the second watchband 33. For example, as shown in FIG. 9, the thin-film sensor 20 is disposed on an inner side of the first watchband 32. Certainly, the thin-film sensor 20 may alternatively be disposed on an inner side of the second watchband 33 (not shown in the figure).

At least one of the first watchband 32 and the second watchband 33 may be connected to the watch body 31 through adhesive bonding, hot melting, a buckle, or the like.

Specific materials of the first watchband 32 and the second watchband 33 are not limited herein. For example, a material of at least one of the first watchband 32 and the second watchband 33 may include but is not limited to a polymer or hydrate material like fluorine rubber, a thermoplastic elastomer, or silica gel.

In this embodiment of this application, the watch buckle 60 is configured to enable one of the first watchband 32 and the second watchband 33 to move relative to the second watchband 33, so that the inner diameter of the ring-shaped structure enclosed by the binding component 30 becomes larger or smaller, to apply pressure to the measured part. For example, the second end of the first watchband 32 is connected to the watch buckle 60, the second end of the second watchband 33 is in transmission connection to the watch buckle 60, and the watch buckle 60 is configured to enable the second end of the second watchband 33 to move relative to the watch buckle 60, so that the inner diameter of the ring-shaped structure enclosed by the pressurization component 10 becomes larger or smaller.

How the watch buckle 60 enables the second end of the second watchband 33 to move relative to the watch buckle 60 is not limited herein. For example, the watch buckle 60 includes a drive motor (not shown in the figure) and a transmission component (not shown in the figure), the second watchband 33 includes a rack (not shown in the figure), and a motor shaft of the drive motor is in transmission connection to the rack via the transmission component. When the drive motor rotates, the transmission component may drive the second watchband 33 to move relative to the first watchband 32, so that the inner diameter of the ring-shaped structure enclosed by the pressurization component 10 becomes larger or smaller, to implement tightening and loosing.

It should be noted that, in addition to the drive motor and the transmission component, the watch buckle 60 may further include at least one of components such as a watch buckle housing (not shown in the figure), a battery (not shown in the figure), a communication module (not shown in the figure), and a control board (not shown in the figure).

The watch buckle housing is configured to accommodate the drive motor, the transmission component, the battery, the communication module, and the control board. A material of the watch buckle housing may be metal alloy like stainless steel, aluminum alloy, zinc alloy, magnesium alloy, or titanium alloy, or may be a polymer material like polyamide, polycarbonate, polyoxymethylene, polybutylene terephthalate, or polyphenylene oxide.

The battery may be a non-rechargeable small zinc-manganese dry battery, a lithium-manganese battery, or an alkaline button battery, or may be a rechargeable lithium-ion battery. If the battery is the rechargeable lithium-ion battery, the watch buckle 60 further includes a charging interface or a charging coil. The battery is connected to the charging interface or the charging coil, and the battery is charged through the charging interface or the charging coil.

The communication module is configured to receive an external instruction, to control a rotation direction and a rotation speed of a rotating shaft of the drive motor, to implement a function of fast/slow tightening/loosing.

The control board is configured to: receive an instruction signal transmitted by the communication module, process the instruction signal into a corresponding motor operating voltage signal, and transmit the motor operating voltage signal to the drive motor. In addition, the control board may also implement a voltage and current modulation function of the battery, so that the battery can stably supply power to each module.

Still refer to FIG. 9. The connection line 40 is disposed on the inner side of the first watchband 32 and is configured to be disposed between the measured part and the first watchband 32. Certainly, the connection line 40 may alternatively be disposed inside the first watchband 32.

It may be understood that, when the thin-film sensor 20 is disposed on the inner side of the second watchband 33, the connection line 40 may alternatively be disposed on the inner side of the second watchband 33 (not shown in the figure) or inside the second watchband 33 (not shown in the figure).

It should be noted that the electronic device shown in FIG. 9 may further include the thin-film component 70 (not shown in the figure). For how to dispose the thin-film component 70, refer to the descriptions in FIG. 7 and FIG. 8. In addition, the rigidness of the packaging component 80 in FIG. 9 or rigidness of the second watchband 33 may be greater than the rigidness of the thin-film sensor 20, so that the thin-film component 70 is removed.

It should be further noted that, in FIG. 9, because the electronic device is the smart watch, the binding component 30 includes the first watchband 32, the second watchband 33, and the watch body 31. However, the binding component 30 may alternatively be of another structure. In some possible implementations, the binding component 30 may alternatively be a watchband component (not shown in the figure), two opposite ends of the watchband component are separately connected to the watch buckle 60, and the watch buckle 60 is configured to enable one end of the watchband component to move relative to the other end of the watchband component, so that the inner diameter of the ring-shaped structure enclosed by the pressurization component 10 becomes larger or smaller.

In the descriptions of embodiments of this application, it should be noted that, unless otherwise clearly specified and limited, terms "mounted", "connected", and "connection" should be understood in a broad sense. For example, the terms may be used for a fastened connection, may be an indirect connection through an intermediate medium, may be an internal connection between two elements, or an interaction relationship between two elements. A person of ordinary skill in the art may understand specific meanings of the foregoing terms in embodiments of this application based on specific cases.

An apparatus or element in embodiments of this application or an implied apparatus or element needs to have a specific position and be constructed and operated in a specific position, and therefore cannot be construed as a limitation on embodiments of this application. In the descriptions of embodiments of this application, unless otherwise precisely and specifically specified, "a plurality of" means two or more.

In the specification, claims, and accompanying drawings of embodiments of this application, terms "first", "second", "third", "fourth", and so on (if any) are intended to distinguish between similar objects but do not necessarily indicate a specific order or sequence. It may be understood that the data used in such a way is interchangeable in proper circumstances, so that embodiments of this application described herein can be implemented in an order other than the order illustrated or described herein. In addition, terms "include", "have" and any other variants are intended to cover non-exclusive inclusion. For example, a process, method, system, product, or device that includes a list of steps or units is not necessarily limited to those expressly listed steps or units, but may include other steps or units not expressly listed or inherent to such a process, method, product, or device.

A term "a plurality of" in this specification means two or more. A term "and/or" in this specification merely describes an association relationship between associated objects, and indicates that three relationships may exist. For example, A and/or B may indicate the following three cases: Only A exists, both A and B exist, and only B exists. In addition, a character "/" in this specification usually indicates an "or" relationship between the associated objects, and a character "/" in a formula usually indicates a "divisible" relationship between the associated objects.

It may be understood that various numbers in embodiments of this application are merely used for distinguishing for ease of description, and are not used to limit the scope of embodiments of this application.

It should be understood that sequence numbers of the foregoing processes do not mean execution sequences in embodiments of this application. The execution sequences of the processes should be determined based on functions and internal logic of the processes, and should not be construed as any limitation on the implementation processes of embodiments of this application.

## Claims

1. A monitoring module, comprising a pressurization component and a thin-film sensor, wherein
the pressurization component is configured to apply pressure to a measured part; and
the thin-film sensor is configured to measure a pulse wave signal at the measured part, and the thin-film sensor is disposed between the pressurization component and the measured part.

2. The monitoring module according to claim 1, wherein the monitoring module further comprises a thin-film component, wherein
the thin-film component is disposed between the pressurization component and the thin-film sensor; or
the thin-film sensor is disposed between the thin-film component and the pressurization component; or
the thin-film component comprises a first part and a second part, the first part is disposed between the pressurization component and the thin-film sensor, and the thin-film sensor is disposed between the first part and the second part.

3. The monitoring module according to claim 1 or 2, wherein the monitoring module further comprises a packaging component, and the thin-film sensor is disposed between the packaging component and the pressurization component and is shielded by the packaging component.

4. The monitoring module according to any one of claims 1 to 3, wherein the pressurization component comprises an inflatable airbag, and the thin-film sensor is disposed between the measured part and the inflatable airbag.

5. The monitoring module according to claim 4, wherein the monitoring module further comprises a binding component, the binding component is configured to limit a ring-shaped structure surrounding the measured part, and the pressurization component is disposed between the binding component and the thin-film sensor.

6. The monitoring module according to claim 4 or 5, wherein the monitoring module further comprises a barometric pressure sensor, and the barometric pressure sensor is configured to monitor pressure in the inflatable airbag.

7. The monitoring module according to any one of claims 1 to 3, wherein the pressurization component comprises a watch buckle and a binding component, a first end of the binding component is fastened to the watch buckle, a second end of the binding component is in transmission connection to the watch buckle, the watch buckle and the binding component are configured to enclose a ring-shaped structure that surrounds the measured part and that has a variable inner diameter, and the thin-film sensor is disposed between the binding component and the measured part.

8. The monitoring module according to any one of claims 1 to 7, wherein the thin-film sensor is of a strip-shaped structure, and a length direction of the thin-film sensor is parallel to a length direction of the pressurization component.

9. The monitoring module according to any one of claims 1 to 8, wherein the thin-film sensor is one of the following: a piezoelectric thin-film sensor, a piezoresistive thin-film sensor, a piezocapacitive thin-film sensor, an ionic-electronic thin-film sensor, or a triboelectric thin-film sensor; or
the thin-film sensor comprises a first electrode layer, a sensing layer, and a second electrode layer that are stacked, one of the first electrode layer and the second electrode layer is disposed between the pressurization component and the sensing layer, and the other is disposed between the sensing layer and the measured part.

10. An electronic device, comprising the monitoring module according to any one of claims 1 to 9.
